# EUROPEAN PATENT APPLICATION

(11) **EP 3 441 104 A1**
(43) Date of publication of application: **13.02.2019**
(21) Application number: 17778910.4
(22) Date of filing: 07.03.2017
(51) Int. Cl.: A61M 25/00

(54) **PERCUTANEOUS CATHETER, AND METHOD FOR PRODUCING PERCUTANEOUS CATHETER TUBE**

(30) Priority: 05.04.2016 JP 2016076097
(71) Applicant: Terumo Kabushiki Kaisha, Tokyo 151-0072 (JP)
(72) Inventor: YOKOYAMA, Kenji, Ashigarakami-gun Kanagawa 259-0151 (JP)
(74) Representative: Casalonga
(86) International application number: PCT/JP2017/009060
(87) International publication number: WO 2017/175531

(57) **Abstract**

[Problem] Provided are a percutaneous catheter in which a kink in a part provided with a side hole can be minimized, and a method of manufacturing a tube for a percutaneous catheter, in which productivity can be improved by forming a side hole by a comparatively easy method.

[Means for Resolution] A percutaneous catheter 30 includes a tubular reinforcement body 320 that includes a plurality of gap portions G and a first opening portion H1, which is formed to have an open area larger than the gap portions, in gaps among a plurality of wires W braided in a reticulated manner; and resin layers 331 and 332 that are provided to coat the reinforcement body and includes a second opening portion H2 which is disposed to overlap the first opening portion. A side hole 63 communicating with a lumen is formed in a part in which the first opening portion and the second opening portion overlap each other.

## Description

### Technical Field

The present invention relates to a percutaneous catheter and a method of manufacturing a tube for a percutaneous catheter.

### Background Art

In the related art, treatment has been performed by a percutaneous cardiopulmonary support (PCPS) method for performing cardiopulmonary resuscitation during emergency treatment, circulation support, and respiratory support. This percutaneous cardiopulmonary support method is a method of temporarily supporting and substituting a cardiopulmonary function using an extracorporeal circulator.

An extracorporeal circulator includes an extracorporeal circulation circuit constituted of a centrifugal pump, an artificial lung, a blood removing path, a blood feeding path, and the like. The extracorporeal circulator exchanges gas in removed blood and feeds the blood to the blood feeding path.

In an extracorporeal circulation circuit, a catheter (cannula) including a lumen, in which blood circulates, is inserted into a patient to form the blood removing path and the blood feeding path. In order to prevent a kink from being generated when in use, each catheter has been configured to include a reinforcement body, and the reinforcement body is coated with a resin. In addition, the catheter is provided with a side hole communicating with the lumen for blood removing and blood feeding.

Commonly, a side hole of a catheter is formed by providing an opening portion by removing a portion of the reinforcement body in a part of only a resin. However, the part of only a resin has rigidity lower than that of a part including the reinforcement body. Therefore, there is concern that a kink will be generated. In addition, it is possible to consider that a kink is prevented by increasing the thickness of a resin. However, if the thickness increases, the outer diameter of a catheter increases. Thus, the performance of being inserted into a living body deteriorates.

For example, PTL 1 discloses a method of disposing a reinforcement plate that is provided separately from a reinforcement body and includes an opening portion, and forming a side hole in the opening portion.

### Citation List

### Patent Literature

PTL 1: Pamphlet of International Publication No. 2009/035745

### Summary of Invention

### Technical Problem

However, a step of providing a reinforcement plate between reinforcement bodies is further required. Therefore, a manufacturing step becomes complicated and a long manufacturing time is required, so that productivity deteriorates. In addition, only the part provided with the reinforcement plate has rigidity different from those of other parts. Therefore, there is concern that a kink will be generated due to the difference in rigidity between the part and other parts.

An object of the present invention is to provide a percutaneous catheter in which a kink in a part provided with a side hole can be minimized, and a method of manufacturing a tube for a percutaneous catheter, in which productivity can be improved by forming a side hole by a comparatively easy method.

### Solution to Problem

In order to achieve the object described above, there is provided a percutaneous catheter including a lumen in which blood circulates. The percutaneous catheter includes a tubular reinforcement body that includes a plurality of gap portions and a first opening portion, which is formed to have an open area larger than the gap portions, in gaps among a plurality of wires braided in a reticulated manner; and a resin layer that is provided to coat the reinforcement body and includes a second opening portion which is disposed to overlap the first opening portion. The percutaneous catheter is constituted by forming the side hole communicating with the lumen in the part in which the first opening portion and the second opening portion overlap each other.

In order to achieve the object described above, there is provided a method of manufacturing a tube used for a percutaneous catheter including a lumen in which blood circulates. The method includes a step of forming a tubular reinforcement body including a plurality of gap portions and a first opening portion, which is formed to have an open area larger than the gap portions, in gaps among a plurality of wires formed in a reticulated manner; a step of forming a resin layer by coating the entirety of the reinforcement body with a resin; and a step of forming a second opening portion by boring the resin layer formed in the first opening portion and forming a side hole, which communicates with the lumen, in a part in which the first opening portion and the second opening portion overlap each other.

### Advantageous Effects of Invention

According to the percutaneous catheter having a configuration as described above, the side hole is formed in the gaps among the wires of the tubularly formed reinforcement body. Therefore, a part reinforced by the wires is formed around the side hole. A part in the vicinity of the side hole has comparatively high rigidity. Therefore, a kink in the part provided with the side hole can be minimized.

According to the method of manufacturing a tube for a percutaneous catheter having a configuration as described above, the side hole can be formed by utilizing the gaps among the wires of the reinforcement body. There is no need to remove the reinforcement body in order to provide the side hole, and to provide another member. Therefore, manufacturing becomes comparatively easy. Accordingly, a manufacturing time can be shortened, so that productivity can be improved.

### Brief Description of Drawings

[Fig. 1] Fig. 1 is a system diagram illustrating an example of an extracorporeal circulator in which a percutaneous catheter according to the embodiment of the present invention is applied.
[Fig. 2] Fig. 2 is a side view illustrating a percutaneous catheter and a dilator according to a first embodiment.
[Fig. 3] Fig. 3 is a sectional side view illustrating the percutaneous catheter according to the first embodiment.
[Fig. 4] Fig. 4 is a side view illustrating a situation in which the dilator is inserted into the percutaneous catheter according to the first embodiment.
[Fig. 5] Fig. 5(A) is an enlarged view of a side hole of the percutaneous catheter, and Fig. 5(B) is a sectional view taken along line E-E in Fig. 5(A).
[Fig. 6] Fig. 6 is an enlarged sectional view illustrating the percutaneous catheter into which the dilator is inserted.
[Fig. 7] Fig. 7 is a view illustrating a distal tip.
[Fig. 8] Fig. 8 is a flow chart for describing a method of manufacturing a tube for a percutaneous catheter according to the first embodiment.
[Fig. 9] Figs. 9(A) to 9(E) are views illustrating the method of manufacturing a tube for a percutaneous catheter according to the first embodiment.
[Fig. 10] Fig. 10 is a flow chart for describing a modification example of the method of manufacturing a tube for a percutaneous catheter according to the first embodiment.
[Fig. 11] Figs. 11(A) to 11(D) are views illustrating a modification example of the method of manufacturing a tube for a
[Fig. 12] Fig. 12(A) is a side view illustrating a distal side of a percutaneous catheter according to a second embodiment, and Fig. 12(B) is a side view illustrating a situation in which a dilator is inserted into the percutaneous catheter according to the second embodiment.
[Fig. 13] Fig. 13 is a plan view illustrating a percutaneous catheter and a dilator according to a third embodiment. [Fig. 14] Fig. 14 is a sectional side view illustrating the percutaneous catheter according to the third embodiment.
[Fig. 15] Fig. 15 is a plan view illustrating a situation in which the dilator is inserted into the percutaneous catheter according to the third embodiment.

### Description of Embodiments

Hereinafter, with reference to the drawings, embodiments of the present invention will be described. The following description does not limit the technical scope or the meaning of the terms disclosed in Claims. In addition, for the convenience of description, there are cases where the dimensional ratios of the drawings are exaggerated and are different from the actual ratios.

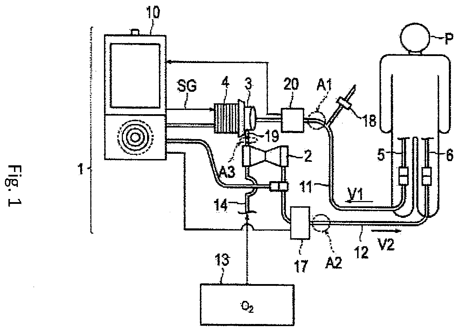

Fig. 1 is a system diagram illustrating an example of an extracorporeal circulator 1 in which a percutaneous catheter according to the embodiments of the present invention is applied and which is used in a percutaneous cardiopulmonary support (PCPS) method for temporarily supporting and substituting the functions of the heart and lungs until the cardiac function recovers when the heart of a patient is weak.

According to the extracorporeal circulator 1, a pump is operated to remove blood from the vein (vena cava) of a patient, and the blood is oxygenated by exchanging gas in the blood using an artificial lung 2. Thereafter, it is possible to perform a veno-arterial (VA) technique of returning the blood to the artery (aorta) of the patient again. The extracorporeal circulator 1 is a device for assisting the heart and lungs. Hereinafter, a technique, in which after blood is removed from a patient and is subjected to predetermined treatment outside the body, the blood is fed into the body of the patient again, will be referred to as "extracorporeal circulation".

As illustrated in Fig. 1, the extracorporeal circulator 1 has a circulation circuit which causes blood to circulate. The circulation circuit includes the artificial lung 2, a centrifugal pump 3, a drive motor 4 which is driving means for driving the centrifugal pump 3, a vein side catheter (percutaneous catheter for blood removing) 5, an artery side catheter (catheter for blood feeding) 6, and a controller 10 which serves as a control unit.

The vein side catheter (catheter for blood removing) 5 is inserted through the femoral vein, and a distal end of the vein side catheter 5 indwells in the right atrium via the inferior vena cava. The vein side catheter 5 is connected to the centrifugal pump 3 via a blood removing tube (blood removing line) 11. The blood removing tube 11 is a conduit line for feeding blood.

The artery side catheter (catheter for blood feeding) 6 is inserted through the femoral artery.

When the drive motor 4 operates the centrifugal pump 3 in response to a command SG of the controller 10, the centrifugal pump 3 removes blood through the blood removing tube 11 and causes the blood to pass through the artificial lung 2. Thereafter, the centrifugal pump 3 can cause the blood to return to a patient P via a blood feeding tube (blood feeding line) 12.

The artificial lung 2 is disposed between the centrifugal pump 3 and the blood feeding tube 12. The artificial lung 2 exchanges gas (oxygenation and/or carbon dioxide removal) in blood. The artificial lung 2 is a membrane-type artificial lung, for example. It is particularly preferable to use a hollow fiber membrane-type artificial lung. Oxygen gas is supplied to the artificial lung 2 from an oxygen gas supply section 13 through a tube 14. The blood feeding tube 12 is a conduit line connecting the artificial lung 2 and the artery side catheter 6 to each other.

As the blood removing tube 11 and the blood feeding tube 12, for example, it is possible to use conduit lines made of a synthetic resin, such as a vinyl chloride resin or silicone rubber, which is highly transparent and flexible to be elastically deformable. Blood (liquid) flows in a V1-direction inside the blood removing tube 11, and blood flows in a V2-direction inside the blood feeding tube 12.

In the circulation circuit illustrated in Fig. 1, an ultrasound air bubble detection sensor 20 is disposed in the middle of the blood removing tube 11. A fast clamp 17 is disposed in the middle of the blood feeding tube 12.

In a case where an air bubble is incorporated into the circuit during extracorporeal circulation due to an erroneous operation of a three-way stopcock 18, damage to the tubes, and the like, the ultrasound air bubble detection sensor 20 detects the incorporated air bubble. In a case where the ultrasound air bubble detection sensor 20 detects that an air bubble is present in blood being fed to the inside of the blood removing tube 11, the ultrasound air bubble detection sensor 20 transmits a measurement signal of air bubble detection to the controller 10. The controller 10 sounds an alarm for notification based on this measurement signal, and the controller 10 lowers the rotational frequency of the centrifugal pump 3 or stops the centrifugal pump 3. Moreover, the controller 10 commands the fast clamp 17 such that the fast clamp 17 immediately closes the blood feeding tube 12. Accordingly, an air bubble is stopped from being fed to the inside of the body of the patient P. In this manner, the controller 10 controls the operation of the extracorporeal circulator 1 to prevent an air bubble from being incorporated into the body of the patient P.

A pressure sensor is provided in the tube 11 (12, 19) of the circulation circuit of the extracorporeal circulator 1. For example, the pressure sensor can be mounted in any one or all of a mounting position A1 of the blood removing tube 11, a mounting position A2 of the blood feeding tube 12 of the circulation circuit, and a mounting position A3 of a connection tube 19 connecting the centrifugal pump 3 and the artificial lung 2 to each other. Accordingly, when extracorporeal circulation is performed with respect to the patient P using the extracorporeal circulator 1, the pressure inside the tube 11 (12, 19) can be measured by the pressure sensor. The mounting position of the pressure sensor is not limited to the mounting positions A1, A2, and A3 described above and can be mounted at any position in the circulation circuit.

### <First Embodiment>

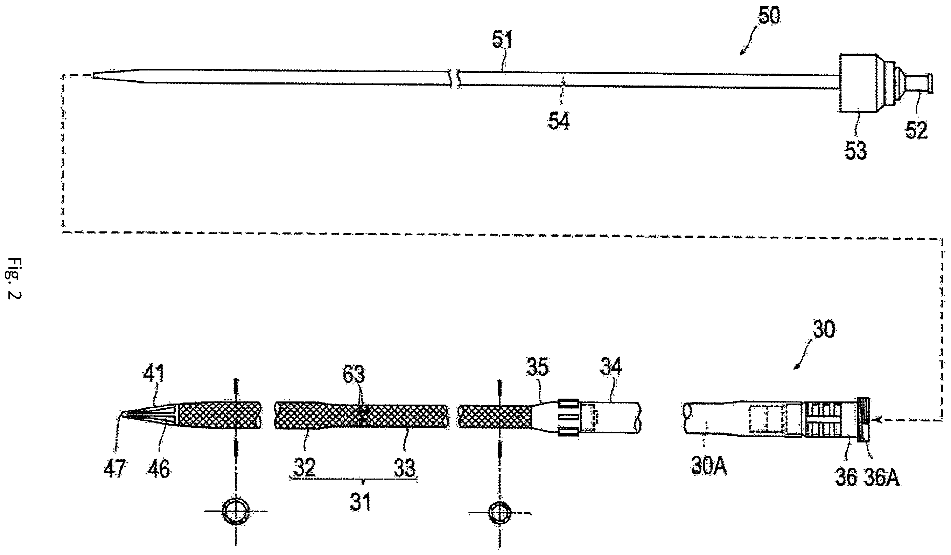

With reference to Figs. 2 to 7, a percutaneous catheter (which will hereinafter be referred to as a "catheter") 30 according to a first embodiment of the present invention will be described. Figs. 2 to 7 are views describing a configuration of the catheter 30 according to the first embodiment. The catheter 30 according to the present embodiment is used as the vein side catheter (catheter for blood removing) 5 in Fig. 1.

As illustrated in Fig. 2, the catheter 30 according to the present embodiment has a catheter tube (corresponding to a tube for a percutaneous catheter) 31 which includes a side hole (corresponding to a side hole on a proximal side) 63, a distal tip 41 which is disposed at a distal end of the catheter tube 31 and includes through-holes 46 and 47, a clamping tube 34 which is disposed on the proximal side of the catheter tube 31, a catheter connector 35 which connects the catheter tube 31 and the clamping tube 34 to each other, and a lock connector 36.

In this specification, a side inserted into a living body will be referred to as a "distal end" or a "distal side", and a hand-side operated by a practitioner will be referred to as a "proximal end" or a "proximal side". A distal portion denotes a certain range including the distal end (outermost distal end) and a surrounding area thereof, and a proximal portion denotes a certain range including the proximal end (innermost proximal end) and a surrounding area thereof.

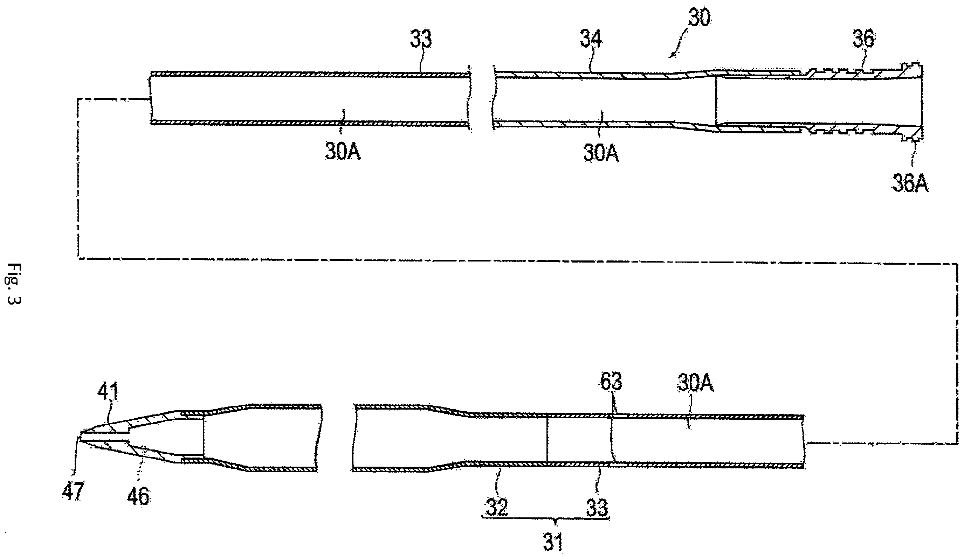

As illustrated in Fig. 3, the catheter 30 has a lumen 30A which is penetrated from the distal end to the proximal end. The through-holes 46 and 47 included in the distal tip 41, and the side hole 63 included in the catheter tube 31 are configured to be disposed in different blood removing targets inside a living body and to be capable of efficiently removing blood.

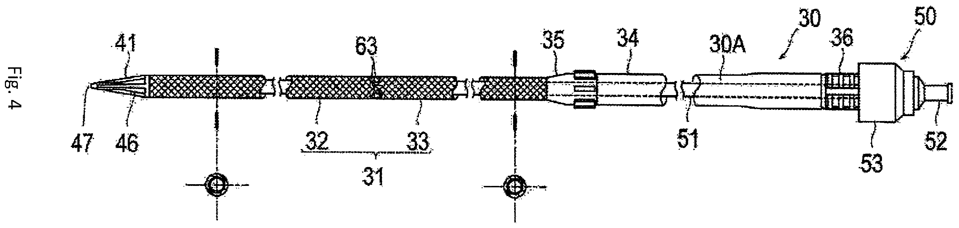

As illustrated in Fig. 4, when the catheter 30 is inserted into a living body, a dilator 50 is used. The dilator 50 is inserted through the lumen 30A of the catheter 30, and the catheter 30 and the dilator 50 are inserted into a living body in a state of being integrated in advance. A method of using the catheter 30 will be described below in detail.

Hereinafter, each of the configurations of the catheter 30 will be described.

As illustrated in Fig. 2, the catheter tube 31 has a first tube 32 and a second tube 33 disposed on the proximal side of the first tube 32. The first tube 32 is configured to have higher flexibility than that of the second tube 33.

In addition, the first tube 32 is configured to have an outer diameter and an inner diameter greater than those of the second tube 33. The first tube 32 and the second tube 33 are integrally constituted and are constituted to have a substantially uniform thickness.

The lengths of the first tube 32 and the second tube 33 are constituted to be lengths necessary for the through-holes 46 and 47 of the distal tip 41, and the side hole 63 to be disposed in desired blood removing targets.
The length of the first tube 32 ranges from 20 to 40 cm, for example, and the length of the second tube 33 ranges from 20 to 30 cm, for example.

The side hole 63 is a hole penetrating a side surface of the second tube 33 and is open for communication between the lumen 30A of the catheter 30. The side hole 63 functions as a hole for blood removing. It is preferable to have a plurality of side holes 63 in a circumferential direction. In the present embodiment, four side holes 63 are provided in the circumferential direction. Accordingly, even if one side hole 63 is adsorbed against a vascular wall and is blocked when blood is removed through the side hole 63, blood can be removed through the remaining side holes 63. Therefore, extracorporeal circulation can be stably performed.

The side hole 63 is a substantially circular hole, and its diameter can range from 2 to 3 mm, for example. The number of side holes 63 and the diameter thereof are not limited thereto and can be suitably set as necessary. In addition, the shape of the side hole 63 is not also limited to a substantially circular shape. For example, the side hole 63 may be formed in a substantially elliptical shape as in a third embodiment, which will be described below.

In the present embodiment, there are two blood removing targets, that is, the right atrium and the inferior vena cava. The catheter 30 is inserted into a living body and indwells therein, such that the through-holes 46 and 47 of the distal tip 41 are disposed in the right atrium and the side hole 63 is disposed in the inferior vena cava.

In a state where the through-holes 46 and 47 and the side hole 63 are disposed in the blood removing targets, the first tube 32 is disposed in the inferior vena cava which is a comparatively wide blood vessel, and the second tube 33 is disposed in the femoral vein which is a comparatively narrow blood vessel.

In addition, when the dilator 50 is inserted through the lumen 30A of the catheter 30, as illustrated in Fig. 4, the first tube 32 having higher flexibility expands in an axial direction so that the outer diameter and the inner diameter of the first tube 32 are reduced. In this state, the outer diameter and the inner diameter of the first tube 32 become substantially the same as the outer diameter and the inner diameter of the second tube 33. The catheter 30 is inserted into a living body in a state where the first tube 32 expands in the axial direction and is reduced in outer diameter. Therefore, the catheter 30 can be inserted in a relatively noninvasive manner.

After the catheter 30 indwells inside a living body, when the dilator 50 is withdrawn from the lumen 30A of the catheter 30, as illustrated in Fig. 2, the first tube 32 contracts in the axial direction so that the outer diameter and the inner diameter of the first tube 32 radially increase. Here, since the first tube 32 is disposed in the inferior vena cava which is a comparatively wide blood vessel, the first tube 32 can be increased in outer diameter.

Here, a pressure loss inside the first tube 32 is the product of the entire length × the (average) cross-sectional area of a passage of the first tube 32. That is, the pressure loss inside the first tube 32 is reduced by increasing the inner diameter of the first tube 32. If the pressure loss inside the first tube 32 is reduced, the flow rate of blood flowing in the circulation circuit increases. Therefore, in order to obtain a sufficient circulation amount of blood, the inner diameter of the first tube 32 needs to be sufficiently increased.

On the other hand, in a case where the thickness is substantially uniform, if the inner diameters of the first tube 32 and the second tube 33 are increased, the outer diameters thereof also increase. Accordingly, a significant load would be applied to a patient when the catheter 30 is inserted into a living body, thereby hindering a low invasive technique.

From the viewpoints described above, the inner diameter of the first tube 32 can range from 9 to 11 mm, for example, and the inner diameter of the second tube 33 can range from 4 to 8 mm, for example. In addition, the thicknesses of the first tube 32 and the second tube 33 can range from 0.3 to 0.5 mm, for example.

In addition, as illustrated in Figs. 2 and 3, it is preferable that each of the distal portion and the proximal portion of the first tube 32 forms a tapered portion which is gradually tapered toward the outer side from the middle of the first tube 32. Accordingly, the inner diameters of the distal end and the proximal end of the first tube 32 are configured to lead to the inner diameter of the distal tip 41 disposed on the distal side and the inner diameter of the second tube 33 disposed on the proximal side, respectively.

Hereinafter, a specific configuration of the catheter tube 31 will be described.

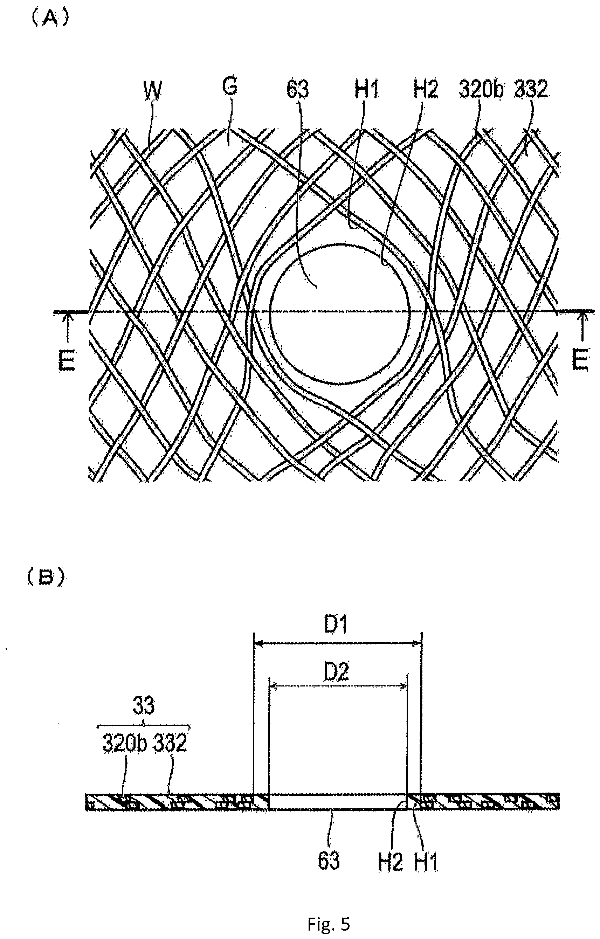

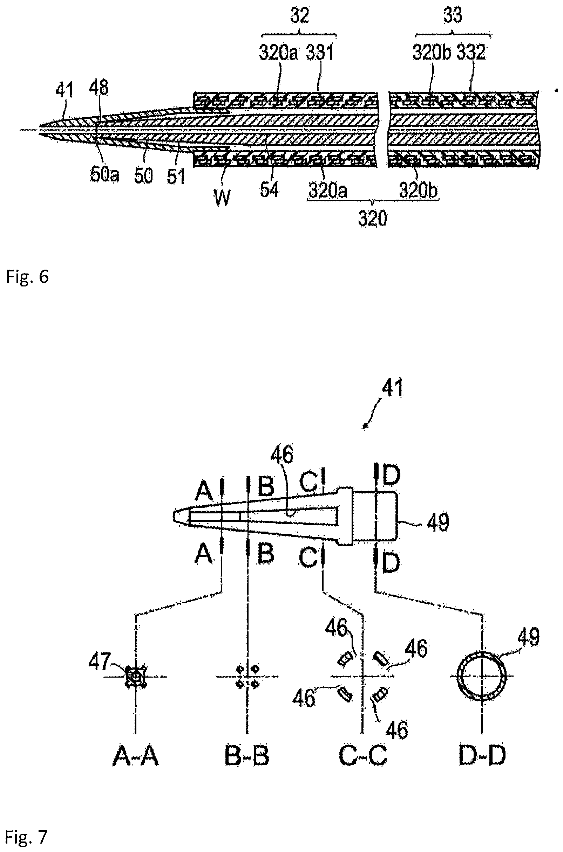

As illustrated in Fig. 6, the catheter tube 31 has a tubular reinforcement body 320 in which wires W are braided to intersect each other in a meshed shape (refer to Fig. 5 (A)), and a first resin layer 331 and a second resin layer 332 which are provided to coat the reinforcement body 320.

The first tube 32 is constituted of a distal portion 320a of the reinforcement body 320 and the first resin layer 331, and the second tube 33 is constituted of a proximal portion 320b of the reinforcement body 320 and the second resin layer 332.

As illustrated in Fig. 5 (A), the reinforcement body 320 includes a plurality of gap portions G and a first opening portion H1, which is formed to have an open area larger than the gap portions G, in gaps among a plurality of wires W braided in a meshed shape.

Here, an "open area of the reinforcement body 320" denotes an area of a closed section surrounded by the plurality of wires W when the catheter 30 seen from the outside in a radial direction. In addition, in a part in which the plurality of wires W overlap each other in a thickness direction, a wire W on the innermost side forms a closed section.

The second resin layer 332 includes a second opening portion H2 which is disposed to overlap the first opening portion H1 of the reinforcement body 320. The side hole 63 is formed in a part in which the first opening portion H1 and the second opening portion H2

In the present embodiment, as illustrated in Fig. 5(B), a maximum length D1 of the first opening portion H1 of the reinforcement body 320 is configured to be longer than a maximum length D2 of the second opening portion H2 of the second resin layer 332. A "maximum length of an opening portion" denotes a length corresponding to the maximum distance between any two points at an outer circumferential edge forming an opening portion. In a case where the second opening portion H2 is formed in a substantially circular shape as in the present embodiment, the maximum length of the opening portion is the diameter of the circle.

In addition, the second opening portion H2 is formed on the inner side of the first opening portion H1. Therefore, the part in which the first opening portion H1 and the second opening portion H2 overlap each other is equivalent to the second opening portion H2.

According to the configuration described above, the second resin layer 332 is formed to cover an inner peripheral surface of the first opening portion H1 of the reinforcement body 320. Therefore, the wires are prevented from being exposed to the inner peripheral surface of the side hole 63.

The second opening portion H2 forms the outer circumferential edge of the side hole 63. That is, the side hole 63 has the same shape as that of the second opening portion H2 and is formed in a substantially circular shape in the present embodiment. The shape of the first opening portion H1 is substantially similar to the shape of the second opening portion H2 and is formed in a substantially circular shape in the present embodiment. The shape of the first opening portion H1 is not limited to a substantially circular shape. The first opening portion H1 may be formed in a substantially elliptical shape as in the third embodiment described below. The maximum length D1 of the first opening portion H1 may range from 2.4 to 3.4 mm, for example.

The wires W forming the reinforcement body 320 are preferably comprised of a shape memory material, such as a known shape memory metal or a known shape memory resin. As a shape memory metal, for example, a titanium-based alloy (Ni-Ti, Ti-Pd, Ti-Nb-Sn, or the like) or a copper-based alloy can be used. In addition, as a shape memory resin, for example, an acryl-based resin, a trans-isoprene polymer, polynorbornene, styrene-butadiene copolymer, and polyurethane can be used.

In the present embodiment, a cross-sectional shape of the wires W constituting the reinforcement body 320 is a rectangular shape. However, the cross-sectional shape is not limited thereto and may be a square shape, a circular shape, or an elliptical shape. In a case where the cross-sectional shape is a circular shape, the wire diameter of the wires W can range from 0.1 mm to 0.2 mm, for example.

The first resin layer 331 constituting the first tube 32 is formed of a material softer than that of the second resin layer 332 constituting the second tube 33. According to this configuration, compared to the second tube 33, the first tube 32 can be soft and highly flexible.

As a material constituting the first resin layer 331, a known resin which is comparatively soft can be used. For example, urethane, polyurethane, silicon, and vinyl chloride having low hardness can be used. In addition, as a material constituting the second resin layer 332, for example, urethane, polyurethane, silicon, and vinyl chloride having high hardness can be used.

In a case where urethane or polyurethane is used, an outer surface may preferably be subjected to hydrophilic coating. Accordingly, the surface of the catheter tube 31 has high lubricity. Therefore, the catheter tube 31 is easily inserted into a living body, operability is improved, and damage to a vascular wall can be prevented. In addition, blood or protein is unlikely to adhere, so that it is possible to expect to prevent a thrombus from being formed.

The distal tip 41 is fixed to the distal end of the first tube 32. As illustrated in Fig. 7, the distal tip 41 has a tapered shape gradually reduced in diameter toward the distal side. The distal tip 41 has a base portion 49 to be inserted into the distal end of the first tube 32, a plurality of through-holes 46 provided on the side surface, and the through-hole 47 provided at the distal end of the distal tip 41. The through-holes 46 and 47 function as holes for blood removing. The through-hole 47 of the distal tip 41 communicates with the lumen 30A of the catheter 30. For example, the distal tip 41 can be formed of a hard plastic.

In addition, the first tube 32 can be effectively prevented from being squashed at the time of blood removal by fixing a hard distal tip 41 to the distal portion of the first tube 32.

As illustrated in Fig. 6, a flat receiving surface 48, which is attached to a flat surface 50a of the dilator 50 used before the catheter 30 is inserted into a living body, is formed on the inner side of the distal tip 41.

The clamping tube 34 is provided on the proximal side of the second tube 33. A lumen through which the dilator 50 can be inserted is provided on the inner side of the clamping tube 34. The clamping tube 34 can be formed by using a material similar to that of the catheter tube 31.

The catheter connector 35 connects the second tube 33 and the clamping tube 34 to each other. A lumen through which the dilator 50 can be inserted is provided on the inner side of the catheter connector 35.

The lock connector 36 is connected to the proximal side of the clamping tube 34. A lumen through which the dilator 50 can be inserted is provided on the inner side of the lock connector 36. A male screw portion 36A provided with screw threads is provided on the outer surface of the lock connector 36 on the proximal side.

Next, a configuration of the dilator 50 will be described.

As illustrated in Fig. 2, the dilator 50 has a dilator tube 51 which is provided to extend in the axial direction, a dilator hub 52 to which a proximal end of the dilator tube 51 is fixed, and a screw ring 53 which is provided at a distal end of the dilator hub 52.

The dilator tube 51 is an elongated body extending in the axial direction and having comparatively high rigidity. The dilator tube 51 is configured to have the entire length in the axial direction longer than the entire length of the catheter 30 in the axial direction. The dilator tube 51 includes a guide wire lumen 54 through which a guide wire (not illustrated) can be inserted (refer to Fig. 6). The dilator tube 51 is guided by the guide wire and is inserted into a living body together with the catheter 30. After the catheter 30 indwells inside a living body, the dilator tube 51 is withdrawn from the catheter 30 by drawing out the dilator hub 52 to the proximal side.

As illustrated in Fig. 6, the distal end of the dilator tube 51 includes the flat surface 50a attached to the receiving surface 48 of the distal tip 41. The dilator tube 51 has comparatively high rigidity and has resilience such that a thrusting force by an operation at hand to the distal side can be transmitted to the distal tip 41. Therefore, the dilator tube 51 plays a role in widening a narrow blood vessel by causing the flat surface 50a to be attached to the receiving surface 48 of the distal tip 41 such that the distal tip 41 is thrust to the distal side.

The screw ring 53 has a female screw portion (not illustrated) in which a screw groove is provided on the inner surface of a lumen. The dilator 50 is configured to be attachable with respect to the catheter 30 by screwing the female screw portion of the screw ring 53 to the male screw portion 36A of the lock connector 36.

### (Method of Manufacturing Catheter Tube)

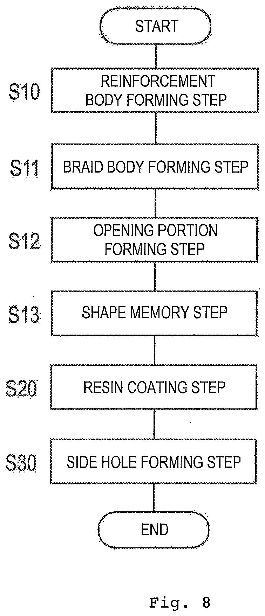

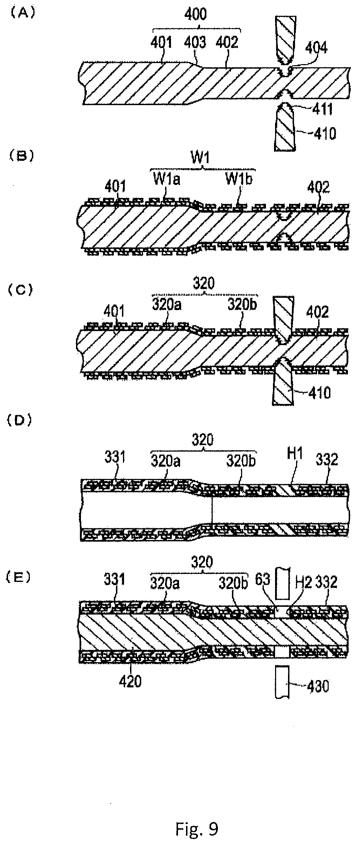

Hereinafter, a method of manufacturing the catheter tube 31 according to the first embodiment will be described with reference to Figs. 8 and 9. Figs. 8 and 9 are views illustrating the method of manufacturing the catheter tube 31 according to the present embodiment.

As illustrated in Fig. 8, the method of manufacturing the catheter tube 31 according to the first embodiment generally includes a step of forming the reinforcement body 320 including the first opening portion H1 (Step S10), a step of coating the reinforcement body 320 with a resin (Step S20), and a step of forming the side hole 63 (Step S30). Hereinafter, each of the steps will be described in detail.

First, as illustrated in Fig. 9(A), a columnar core bar 400 extending in the axial direction and a pin 410 tapered in a conical shape are prepared.

The core bar 400 has a columnar first core bar portion 401 having a uniform diameter, a columnar second core bar portion 402 formed to have a cross-sectional diameter smaller than that of the first core bar portion 401, and a tapered portion 403 formed to have a diameter which is gradually reduced. The first core bar portion 401 and the second core bar portion 402 are integrally formed via the tapered portion 403. The second core bar portion 402 has a female screw portion 404 partially including a screw groove on the side surface.

The tapered distal end of the pin 410 is configured to have a male screw portion 411 including a screw thread so as to be able to be screwed into the female screw portion 404 of the core bar 400.

Next, the reinforcement body 320 including the first opening portion H1 is formed (Step S10). With reference to Fig. 8, the step of forming the reinforcement body 320 (Step S10) includes a step of forming a braid body W1 by braiding the wires W (Step S11), a step of forming the first opening portion H1 in the braid body W1 (Step S12), and a step of shape-memorizing the braid body W1 by heating the braid body W1 (Step S13).

First, as illustrated in Fig. 9(B), the meshed braid body W1, in which the wires W are braided by a braiding machine (not illustrated) to intersect each other on the outer surface of the core bar 400 and which includes a plurality of gaps, is formed (Step S11). In the braid body W1, a distal portion W1a formed by braiding the wires W in the first core bar portion 401 is formed to have an inner diameter greater than that of a proximal portion W1b formed by braiding the wires W in the second core bar portion 402.

Next, as illustrated in Fig. 9(C), the pin 410 is inserted into a portion of the gaps in the proximal portion W1b of the braid body W1 disposed in the second core bar portion 402, and the first opening portion H1 is formed by widening the gap (Step S12). Specifically, a male screw portion of the pin 410 is screwed into the female screw portion 404 of the core bar 400. The gap can be gradually widened by inserting the pin 410 into the gap of the reinforcement body 320 from the tapered distal end. Accordingly, a state where the gap is widened by the pin 410 is formed. The widened gap forms the first opening portion H1. In addition, the plurality of gap portions G are formed in gaps into which the pin 410 is not inserted.

Next, while retaining the state where the expanded gap is widened by insertion of the pin 410, shape-memorization is performed by heating the braid body W1 (Step S13). The heating temperature can be set to range from 400°C to 500°C, for example.

After the braid body W1 is subjected to shape-memorizing, the pin 410 is removed from the core bar 400, and the shape-memorized braid body W1 is detached from the core bar 400, thereby completing the reinforcement body 320. Accordingly, the plurality of gap portions G and the first opening portion H1 having an open area larger than the gap portions G are formed in the gaps among the wires W. The distal portion 320a of the reinforcement body 320 corresponding to the distal portion W1a of the braid body W1 is formed to have an inner diameter greater than that of the proximal portion 320b of the braid body W1 corresponding to the proximal portion W1b of the braid body W1.

Next, as illustrated in Fig. 9(D), the reinforcement body 320 is coated with a resin, and thus the first resin layer 331 and the second resin layer 332 are formed (Step S20). The first resin layer 331 having flexibility higher than that of the second resin layer 332 is formed in the distal portion 320a of the reinforcement body 320, and the second resin layer 332 is formed in the proximal portion 320b of the reinforcement body 320. A method of forming the first resin layer 331 and the second resin layer 332 in the reinforcement body 320 is not particularly limited. However, the first resin layer 331 and the second resin layer 332 can be formed by an immersion method (dipping method) or insert molding, for example. In the present embodiment, a method using the immersion method will be described as an example.

In the immersion method, a solution including a resin is applied to the reinforcement body 320, and the applied solution is dried, thereby forming the first resin layer 331 and the second resin layer 332 on a surface of the reinforcement body 320. First, the distal portion 320a of the reinforcement body 320 is immersed in the solution including a resin. The immersion time is not particularly limited. The immersion time can range from 5 seconds to 30 minutes, for example. The reinforcement body 320 may be immersed in a solution a plurality of times in a divided manner. After the solution is applied, the solution is dried and the first resin layer 331 is formed in the distal portion 320a of the reinforcement body 320. The drying temperature and the drying time are not particularly limited. However, the drying temperature preferably ranges from 20°C to 80°C, and the drying time preferably ranges from 15 minutes to 5 hours. Examples of a device used for drying the solution include an oven, a dryer, and a microwave heating device. Similarly, the second resin layer 332 is also formed in the proximal portion 320b of the reinforcement body 320.

Next, as illustrated in Fig. 9(E), a part of the second resin layer 332 disposed in the first opening portion H1 of the reinforcement body 320 is bored, and thus the side hole 63 is formed (Step S30). Specifically, first, a core rod 420 is inserted into the lumen of the reinforcement body 320 in which the first resin layer 331 and the second resin layer 332 are formed. Next, a part of the second resin layer 332, in which the first opening portion H1 of the reinforcement body 320 is disposed, is punched with a punch 430. At this time, the second opening portion H2 is formed to be smaller than the first opening portion H1 by using the punch 430 having a diameter smaller than that of the first opening portion H1 of the reinforcement body 320. Accordingly, as illustrated in Fig. 5(B), the second resin layer 332 is formed to cover the inner peripheral surface of the first opening portion H1 of the reinforcement body 320. Therefore, the wires W can be prevented from being exposed from the inner peripheral surface of the side hole 63.

Lastly, the core rod 420 is pulled out, thereby obtaining the catheter tube 31 in which the side hole 63 is formed.

### (Method of Using Catheter)

Next, the method of using the catheter 30 described above will be described. Fig. 2 illustrates a state before the dilator tube 51 of the dilator 50 is inserted through the lumen 30A of the catheter 30, and Fig. 4 illustrates a state after the dilator tube 51 is inserted through the lumen 30A of the catheter 30, respectively.

First, as illustrated in Fig. 4, the dilator tube 51 of the dilator 50 is inserted through the lumen 30A of the catheter 30. The dilator tube 51 passes through the insides of the second tube 33 and the first tube 32 in order, and the flat surface 50a of the dilator tube 51 is attached to the receiving surface 48 of the distal tip 41 (refer to Fig. 6) .

Here, as illustrated in Fig. 2, the entire length of the dilator tube 51 in the axial direction is configured to be longer than the entire length of the catheter 30 in the axial direction. Therefore, in a state where the flat surface 50a of the dilator tube 51 is attached to the receiving surface 48 of the distal tip 41, the distal tip 41 is pressurized to the distal side. Accordingly, the distal end of the first tube 32 fixed to the distal tip 41 is pulled to the distal side.

Accordingly, the catheter 30 receives a force in a direction of expanding, and the first tube 32 having comparatively high flexibility in the catheter 30 expands in the axial direction. In response to the first tube 32 expanding in the axial direction, the outer diameter of the first tube 32 is reduced and becomes substantially the same as the outer diameter of the second tube 33. In a state where the first tube 32 expands in the axial direction, the proximal end of the catheter 30 is fixed to the dilator hub 52.

Next, the catheter 30, through which the dilator tube 51 is inserted, is inserted along a guide wire (not illustrated) which has been inserted into a target site inside a living body in advance. At this time, the outer diameter of the first tube 32 becomes substantially the same as the outer diameter of the second tube 33 in response to the dilator 50 being inserted through the catheter 30. Therefore, the catheter 30 can be inserted into a living body in a low invasive manner. The catheter 30 is inserted into a living body until the through-holes 46 and 47 of the distal tip 41 are disposed in the right atrium and the side hole 63 is disposed in the inferior vena cava, and the catheter 30 indwells therein. In a state where the through-holes 46 and 47 and the side hole 63 are disposed in a blood removing target, the first tube 32 is disposed in the inferior vena cava which is a comparatively wide blood vessel, and the second tube 33 is disposed in the femoral vein which is a comparatively narrow blood vessel.

Next, the dilator tube 51 and the guide wire are withdrawn from the catheter 30. At this time, the dilator tube 51 and the guide wire are temporarily pulled to the location of the clamping tube 34 of the catheter 30 and are clamped by forceps (not illustrated). Thereafter, the dilator tube 51 and the guide wire are completely withdrawn from the catheter 30. Since the dilator tube 51 is withdrawn from the lumen 30A of the catheter 30, the catheter 30 is released from a force of the dilator 50 received in the direction of expanding in the axial direction. Therefore, the first tube 32 contracts in the axial direction, and the outer diameter and the inner diameter of the first tube 32 increase. Accordingly, the pressure loss inside the first tube 32 can be reduced.

Next, the lock connector 36 of the catheter 30 is connected to the blood removing tube 11 of the extracorporeal circulator 1 in Fig. 1. After it is checked that connection of the catheter on the blood feeding side is completed, the forceps on the clamping tube 34 are released, and extracorporeal circulation starts.

When extracorporeal circulation ends, the catheter 30 is withdrawn from the blood vessel, and the insertion location is subjected to hemostatic restoration by a surgical technique as necessary.

As described above, the catheter 30 according to the present embodiment has the tubular reinforcement body 320 that includes the plurality of gap portions G and the first opening portion H1, which is formed to have an open area larger than the gap portions G, in the gaps among the plurality of wires W braided in a reticulated manner; and the second resin layer 332 that is provided to coat the reinforcement body 320 and includes the second opening portion H2 which is disposed to overlap the first opening portion H1. The side hole 63 communicating with the lumen 30A of the catheter 30 is formed in a part in which the first opening portion H1 and the second opening portion H2 overlap each other.

According to the catheter 30 having such a configuration, the side hole 63 is formed in the gaps among the wires W of the tubularly-formed reinforcement body 320. Therefore, a part reinforced by the wires W is formed around the side hole 63. The rigidity of a part in the vicinity of the side hole 63 remains comparatively high. Therefore, it is possible to minimize a kink in a part provided with the side hole 63.

In addition, the first tube 32 is configured to have an inner diameter greater than that of the second tube 33 and to be highly flexible. In addition, the distal tip 41 internally includes the flat receiving surface 48 planarly receiving the flat surface 50a of the distal portion of the dilator 50 inserted through the lumen 30A when the catheter 30 is inserted into a living body. Accordingly, due to the flat surface 50a of the dilator tube 51 attached to the receiving surface 48 of the distal tip 41, the first tube 32 configured to be highly flexible expands in the axial direction and is reduced in outer diameter. Accordingly, a catheter 60 can be inserted into a living body in a low invasive manner. In addition, when the dilator 50 is withdrawn from the catheter 30, the first tube 32 contracts, and the inner diameter of the first tube 32 increases. Accordingly, the pressure loss inside the first tube 32 can be reduced.

In addition, the side hole 63 is disposed in the second tube 33. Accordingly, blood can be removed from not only from the distal side of the catheter 30 but also from the proximal side. Therefore, blood can be efficiently removed.

The method of manufacturing the catheter tube 31 according to the present embodiment includes the step of forming the tubular reinforcement body 320 (Step S10), the step of forming the first resin layer 331 and the second resin layer 332 by coating the reinforcement body 320 with a resin (Step S20), and the step of forming the second opening portion H2 by boring the second resin layer 332 formed in the first opening portion H1 and forming the side hole 63 (Step S30).

According to the method of manufacturing the catheter tube 31 having a configuration as described above, the side hole 63 can be formed by utilizing the gaps among the wires W of the reinforcement body 320. Since there is no need to remove the reinforcement body 320 or to provide another member in order to provide the side hole 63, manufacturing becomes comparatively easy. Accordingly, a manufacturing time can be shortened, so that productivity can be improved.

In addition, the step of forming the reinforcement body 320 (Step S10) includes the step of forming the first opening portion H1 by widening a part of the gaps among the wires W of the braid body W1 (Step S12). The first opening portion H1 can be formed in a comparatively simple manner by widening the gaps among the wires W of the reinforcement body 320. Accordingly, the side hole 63 can be formed in a shorter time, so that productivity can be further improved.

In addition, the step of forming the reinforcement body 320 (Step S10) further includes the step of shape-memorization of the wires W of the braid body W1 by heating the wires W while maintaining the gaps among the wires W in which the first opening portion H1 is formed (Step S13) . Accordingly, the shape of the first opening portion H1 of the reinforcement body 320 can be reliably retained.

### (Modification Example of Method of Manufacturing Catheter Tube)

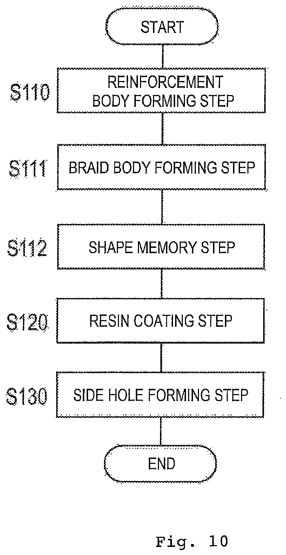

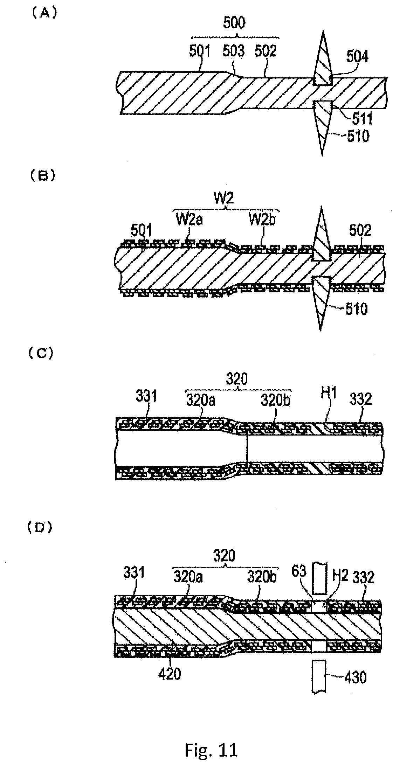

Hereinafter, a modification example of the method of manufacturing the catheter tube 31 according to the first embodiment will be described with reference to Figs. 10 and 11. Figs. 10 and 11 are views illustrating the modification example of the method of manufacturing the catheter tube 31 according to the present embodiment.

With reference to Fig. 10, similar to the first embodiment, the method of manufacturing the catheter tube 31 according to the modification example includes a step of forming the reinforcement body 320 including the first opening portion H1 (Step S110), a step of coating the reinforcement body 320 with a resin (Step S120), and a step of forming the side hole 63 (Step S130). Because only the step of forming the reinforcement body 320 (Step S110) differs from the first embodiment, a detailed description of other steps will be omitted.

The step of forming the reinforcement body 320 (Step S110) includes a step of forming a braid body W2 by braiding the wires W (Step S111), and a step of shape-memorizing the braid body W2 by heating the braid body W2 (Step S112).

First, as illustrated in Fig. 11(A), a columnar core bar (corresponding to a first jig) 500 extending in the axial direction, and a tapered conical pin (corresponding to a second jig) 510 disposed in a part in a direction intersecting the axial direction on an outer peripheral surface of the core bar 500 are prepared.

Similar to the first embodiment, the core bar 500 has a first core bar portion 501, a second core bar portion 502 formed to have a cross-sectional diameter smaller than that of the first core bar portion 501, and a tapered portion 503. The second core bar portion 502 has a female screw portion 504 partially including a screw groove on the side surface.

One end portion of the pin 510 is configured to have a male screw portion 511 including a screw thread so as to be able to be screwed into the female screw portion 504 of the second core bar portion 502. The pin 510 is configured to be attachable to the core bar 500 via the male screw portion 511 and the female screw portion 504.

Next, as illustrated in Fig. 11(B), the meshed braid body W2, in which the wires W are braided by a braiding machine to intersect each other on the outer surface of the second core bar portion 502 to which the pin 510 is fixed and which includes a plurality of gaps, is formed (Step S111). In this state, an enlarged gap, which is formed by braiding the wires W around a part in which the pin 510 is disposed, is formed to have an open area larger than those of other gaps. Accordingly, a state where the gap is widened by the pin 510 is formed. The widened gap forms the first opening portion H1. In addition, the plurality of gap portions G are formed in gaps into which the pin 510 is not inserted.

In addition, a distal portion W2a in the braid body W2 formed by braiding the wires W in the first core bar portion 501 is formed to have an inner diameter greater than that of a proximal portion W2b formed by braiding the wires W in the second core bar portion 502.

Next, while retaining the state where the gap is widened by the pin 510, shape-memorization is performed by heating the braid body W2 (Step S112) . Accordingly, the first opening portion H1 is formed in the gap in the widened state. After the braid body W2 is subjected to shape-memorization, the pin 510 is removed from the core bar 500, and the shape-memorized braid body W2 is detached from the core bar 500, thereby completing the reinforcement body 320.

Thereafter, as illustrated in Fig. 11(C), the reinforcement body 320 is coated with a resin (Step S120), and as illustrated in Fig. 11(D), the second opening portion H2 is formed by boring the second resin layer 332 formed in the first opening portion H1, and thus the side hole 63 is formed (Step S130) . Lastly, the core rod 420 is pulled out, thereby obtaining the catheter tube 31 in which the side hole 63 is formed.

As described above, in the method of manufacturing the catheter tube 31 according to the modification example, the step of forming the reinforcement body 320 (Step S110) includes the step of forming the first opening portion H1 around the pin 510 while braiding the wires W on the outer peripheral surface of the core bar 500. Accordingly, the first opening portion H1 can be formed at the same time as the wires W is braided. Since there is no need to separately provide the step of forming the first opening portion H1, it is possible to reduce a workload and to further improve productivity.

### <Second Embodiment>

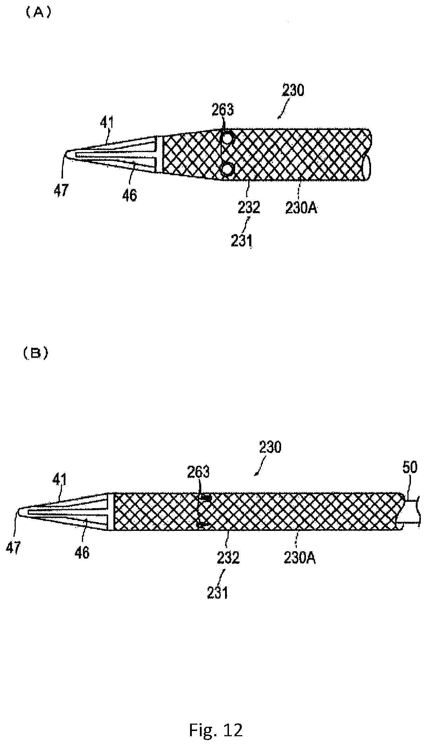

With reference to Fig. 12, a percutaneous catheter (which will hereinafter be referred to as a "catheter") 230 according to a second embodiment of the present invention will be described. Fig. 12(A) is a side view illustrating the distal side of the catheter 230, and Fig. 12(B) is a side view illustrating a situation in which the dilator 50 is inserted into the catheter 230.

Similar to the first embodiment, the catheter 230 according to the second embodiment has a catheter tube (corresponding to a tube for a percutaneous catheter) 231. The catheter tube 231 includes a first tube 232 and the second tube 33 disposed on the proximal side of the first tube 232. The first tube 232 is configured to have higher flexibility than that of the second tube 33.

As illustrated in Fig. 12(A), the catheter 230 differs from the first embodiment in further including a side hole 263 on the distal side in the first tube 232. The same reference signs are applied to portions having the same function as that in the first embodiment, and description thereof is omitted.

The first tube 232 is configured to be highly flexible. Therefore, when the dilator 50 is inserted through a lumen 230A of the catheter 230, the first tube 232 having high flexibility expands in the axial direction. At this time, in response to the first tube 232 expanding in the axial direction, the side hole 263 on the distal side is deformed in a longitudinally elongated manner. Accordingly, as illustrated in Fig. 12(B), the open area of the side hole 263 on the distal side is reduced. The side hole 263 on the distal side may be configured to be completely blocked in response to the first tube 232 expanding in the axial direction.

In addition, similar to the first embodiment, the wires W are formed of a shape memory material. Therefore, when the dilator 50 is withdrawn from the catheter 230 after being disposed in a blood removing target, the wires W disposed around the side hole 263 on the distal side return to the original shape due to a restoring force. Accordingly, the side hole 263 on the distal side is open again.

As described above, the side hole 263 on the distal side of the catheter 230 according to the second embodiment is disposed in the first tube 232. In addition, the wires W are formed of a shape memory material.

According to the catheter 230 having such a configuration, when the dilator 50 is inserted through the catheter tube 231, the first tube 232 expands so that the open area of the side hole 263 on the distal side is reduced. Accordingly, since the open area of the side hole 263 on the distal side is reduced when the catheter 230 is inserted into a living body in a state where the dilator 50 is inserted through, blood can be prevented from being unintentionally removed from the inside of a blood vessel via the side hole 263 on the distal side.

In addition, when the dilator 50 is withdrawn from the catheter 230, the first tube 232 contracts and returns to the original shape, and the side hole 263 on the distal side is open again. Therefore, blood can be favorably removed through the side hole 263 on the distal side.

### <Third Embodiment>

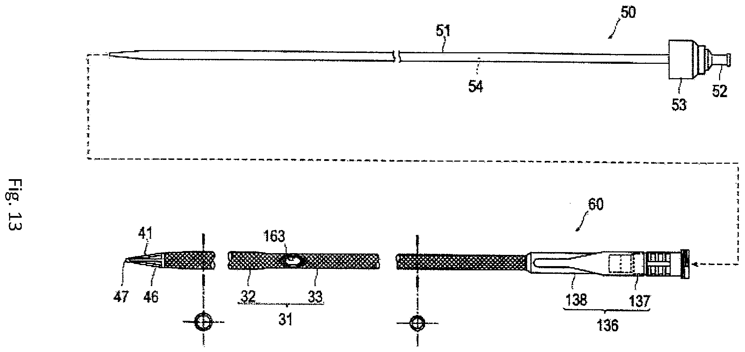

With reference to Figs. 13 to 15, a percutaneous catheter (which will hereinafter be referred to as a "catheter") 60 according to the third embodiment of the present invention will be described. Figs. 13 to 15 are views describing a configuration of the catheter 60 according to the second embodiment.

A catheter 60 is a so-called double lumen catheter, which can perform both blood feeding and blood removing at the same time. Therefore, in the present embodiment, for the extracorporeal circulator 1 in Fig. 1, a technique is performed by using only the catheter 60, without using two catheters, that is, the vein side catheter (catheter for blood removing) 5 and the artery side catheter (catheter for blood feeding) 6.

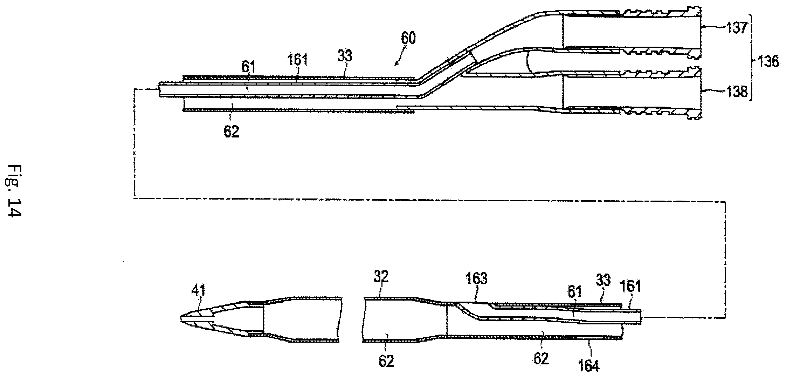

As illustrated in Fig. 14, the catheter 60 according to the present embodiment differs from the catheter 30 according to the first embodiment in that a third tube 161 including a first lumen 61 communicating with a side hole (corresponding to a side hole on the proximal side) 163 for blood feeding has a double tube structure disposed in the lumen of the second tube 33.

According to the catheter 60, the pump of the extracorporeal circulator 1 is operated to remove blood from the vein (vena cava) of a patient, and the blood is oxygenated by exchanging gas in the blood using the artificial lung 2. Thereafter, it is possible to perform a veno-venous (VV) technique of returning the blood to the vein (vena cava) of the patient again.

Hereinafter, each of the configurations of the catheter 60 will be described. Description of parts in common with those in the first embodiment is omitted, and only the characterized portions of the third embodiment will be described. The same reference signs are applied to portions having the same function as that in the first embodiment, and description thereof is omitted.

The catheter 60 has the first tube 32, the second tube 33, the distal tip 41 which is disposed at the distal end of the first tube 32 and includes the through-holes 46 and 47, and the third tube 161 which is disposed in the lumen of the second tube 33.

As illustrated in Fig. 14, the catheter 60 has the first lumen 61 which functions as a blood feeding path, and a second lumen 62 which functions as a blood removing path.

The first lumen 61 is formed in the lumen of the third tube 161. The second lumen 62 is formed in the lumens of the first tube 32 and the second tube 33 and penetrates the lumens from the distal end to the proximal end.

The second tube 33 includes the side hole 163 for blood feeding communicating with the first lumen 61 (blood feeding path), and a side hole (corresponding to a side hole on the proximal side) 164 for blood removing communicating with the second lumen 62 (blood removing path) . The side hole 163 for blood feeding and the side hole 164 for blood removing are configured to have an elliptical shape.

The third tube 161 is inserted into the second lumen 62 from the proximal side of the second tube 33 and is interlocked with the side hole 163 for blood feeding.

The side hole 163 for blood feeding is disposed in a blood feeding target inside a living body. Blood oxygenated by the artificial lung 2 is fed into a living body via the side hole 163 for blood feeding.

The through-holes 46 and 47 and the side hole 164 for blood removing included in the distal tip 41 are configured to be disposed in different blood removing targets inside a living body such that blood can be efficiently removed. In addition, even if the through-holes 46 and 47 or the side hole 164 for blood removing is adsorbed to a vascular wall and is blocked, blood can be removed through a hole which is not blocked. Therefore, extracorporeal circulation can be stably performed.

In the present embodiment, the catheter 60 is inserted through the internal jugular vein of the neck, and the distal end indwells in the inferior vena cava via the upper vena cava and the right atrium. The blood feeding target is the right atrium, and there are two blood removing targets, that is, the upper vena cava and the inferior vena cava.

As illustrated in Fig. 13, in a state where the dilator 50 is inserted, the catheter 60 is inserted into a living body and indwells therein such that the through-holes 46 and 47 of the distal tip 41 are disposed in the inferior vena cava and the side hole 164 for blood removing is disposed in the internal jugular vein.

Similar to the first embodiment, the first tube 32 is configured to have an inner diameter greater than that of the second tube 33. In a state where the through-holes 46 and 47 and the side hole 63 are disposed at the blood removing targets, the first tube 32 is disposed in the inferior vena cava which is a comparatively wide blood vessel, and the second tube 33 is disposed in the femoral vein which is a comparatively narrow blood vessel.

As illustrated in Fig. 14, a lock connector 136 has a first lock connector 137 which communicates with the first lumen 61, and a second lock connector 138 which is provided to be parallel to the first lock connector 137 and communicates with the second lumen 62. The lock connector 136 is a Y-connector having a Y-shape which is formed by the first lock connector 137 bifurcated from the second lock connector 138.

The first lock connector 137 is interlocked with the proximal portion of the third tube 161. The second lock connector 138 is coaxially interlocked with the proximal portion of the second tube 33. A blood feeding tube (blood feeding line) is connected to the first lock connector 137, and a blood removing tube (blood removing line) is connected to the second lock connector 138.

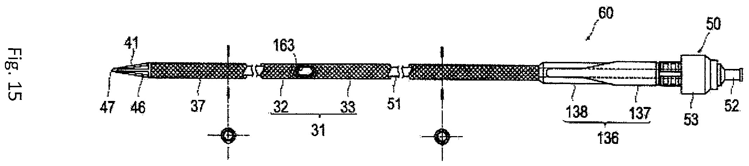

The first tube 32 exhibits the same function as that in the first embodiment and also has an operational effect in common. When the dilator 50 is inserted through the catheter 60, as illustrated in Fig. 15, the first tube 32 expands so that the outer diameter and the inner diameter are reduced. Accordingly, the catheter 60 can be inserted into a living body in a low invasive manner. In addition, when the dilator 50 is withdrawn from the catheter 30, as illustrated in Fig. 13, the first tube 32 contracts so that the inner diameter of the first tube 32 increases. Accordingly, the pressure loss inside the first tube 32 can be reduced.

As described above, according to the catheter 60 of the present embodiment, one catheter can perform both functions of blood removing and blood feeding.

Hereinabove, the percutaneous catheter and the method of manufacturing a tube for a percutaneous catheter according to the present invention have been described through the embodiments and the modification example. The present invention is not limited to only the configurations described in the embodiments and the modification example and can be suitably changed based on Claims.

For example, the second opening portion of the resin layer has been formed on the inner side of the first opening portion of the reinforcement body, and the resin layer has been formed to cover the inner peripheral surface of the first opening portion. However, the configuration is not limited as long as the configuration includes a part in which the first opening portion and the second opening portion overlap each other.

In addition, the number and the disposing location of side

In addition, the first tube has been configured to have flexibility higher than that of the second tube by using different materials for the resin layers constituting the first tube and the second tube. However, the present invention is not limited to the configuration. For example, the first tube and the second tube may be configured to have different flexibility by changing the braid angle, the wire diameter, and the number of wires.

In addition, the present invention is not limited to the configuration in which the resin layer covers the entirety of the reinforcement body. The reinforcement body may have an exposed part. In this case, it is preferable that at least the outer peripheral surface of the tubular reinforcement body is configured to be covered. Accordingly, the reinforcement body is not exposed on a side facing a vascular wall when the catheter is inserted into a living body.

In addition, a material constituting the wires is not limited to a shape memory material as long as the material maintains an open shape of the side hole and has a function of reinforcing the resin layer before the catheter tube expands. For example, the wires can be formed of a known elastic material.

In addition, the first tube and the second tube may be constituted as separate bodies. In this case, the first tube and the second tube may be connected to each other via a connector.

In addition, the catheter according to the first embodiment described above has been used as a catheter for blood removing. However, the present invention is not limited thereto. The catheter according to the first embodiment may be used as a catheter for blood feeding.

In addition, the side hole according to the second embodiment described above has been provided in each of the first tube and the second tube. However, the present invention is not limited thereto. The side hole on the distal side may be provided in only the first tube. In this case, a connector including a side hole may be provided on the proximal side of the first tube.

In addition, in the third embodiment described above, the through-hole at the distal tip has been used for blood removing. However, the present invention is not limited thereto. The through-hole may be used for blood feeding. In this case, the first lumen is used for blood removing, and the second lumen is used for blood feeding.

In addition, in the first embodiment and the third embodiment described above, the first tube has included the first resin layer. However, the present invention is not limited to the configuration. The first tube may be configured to have no first resin layer.

This application is claimed on Japanese Patent Application No. 2016-076097, filed on April 5, 2016, the content of which is incorporated herein by reference.

### Reference Signs List

30, 60, 230 catheter (percutaneous catheter),
30A, 230A lumen,
31, 231 catheter tube (tube for percutaneous catheter),
32, 232 first tube,
33 second tube,
320 reinforcement body,
331 first resin layer (resin layer),
332 second resin layer (resin layer),
63, 163, 164 side hole (side hole on proximal side),
263 side hole (side hole on distal side),
50 dilator,
500 core bar (first jig),
510 pin (second jig),
W wire,
W1, W2 braid body,
G gap portion,
H1 first opening portion,
H2 second opening portion

## Claims

1. A percutaneous catheter including a lumen in which blood circulates, the percutaneous catheter comprising:
a tubular reinforcement body including a plurality of gap portions and a first opening portion, which is formed to have an open area larger than the gap portions, in gaps among a plurality of wires braided in a reticulated manner; and
a resin layer coating the reinforcement body and including a second opening portion which is disposed to overlap the first opening portion,
wherein a side hole communicating with the lumen is formed in a part in which the first opening portion and the second opening portion overlap each other.

2. The percutaneous catheter according to Claim 1, further comprising:
a first tube including the reinforcement body and the resin layer;
a second tube interlocked on a proximal side of the first tube and including the reinforcement body and the resin layer; and
a distal tip fixed to a distal end of the first tube,
wherein the first tube is configured to have an inner diameter greater than the inner diameter of the second tube and to be highly flexible, and wherein the distal tip internally includes a flat receiving surface planarly receiving a distal portion of a dilator inserted through the lumen when the percutaneous catheter is inserted into a living body.

3. The percutaneous catheter according to Claim 2, wherein the side hole includes a side hole disposed on the proximal side of the second tube.

4. The percutaneous catheter according to Claim 2 or 3,
wherein the wires are formed of a shape memory material, and
wherein the side hole includes a side hole disposed on a distal side of the first tube.

5. A method of manufacturing a tube used for a percutaneous catheter including a lumen in which blood circulates, the method comprising:
a step of forming a tubular reinforcement body including a plurality of gap portions and a first opening portion, which is formed to have an open area larger than the gap portions, in gaps among a plurality of wires formed in a reticulated manner;
a step of forming a resin layer by coating the reinforcement body with a resin; and
a step of forming a second opening portion by boring the resin layer formed in the first opening portion and forming a side hole, which communicates with the lumen, in a part in which the first opening portion and the second opening portion overlap each other.

6. The method of manufacturing a tube for a percutaneous catheter according to Claim 5,
wherein the step of forming a reinforcement body includes a step of forming the first opening portion by widening a part of the gaps among the wires.

7. The method of manufacturing a tube for a percutaneous catheter according to Claim 5,
wherein the step of forming a reinforcement body includes a step of forming the first opening portion around a second jig disposed in a part in a direction intersecting an axial direction on an outer peripheral surface of a columnar first jig extending in the axial direction while braiding the wires on the outer peripheral surface of the first jig.

8. The method of manufacturing a tube for a percutaneous catheter according to any one of Claims 5 to 7,
wherein the step of forming a reinforcement body includes a step of shape-memorizing the wires by heating the wires while maintaining the gaps among the wires in which the first opening portion is formed.
